Europäisches Patentamt

European Patent Office

Office européen des brevets

(19)

(11) Publication number: **0 190 814 B1**

# EUROPEAN PATENT SPECIFICATION

(12)

(45) Date of publication of patent specification: **13.05.92**  (51) Int. Cl.⁵: **A61L 15/16**, A61F 13/02

(21) Application number: **86300024.6**

(22) Date of filing: **03.01.86**

(54) **Wound dressing.**

(30) Priority: **04.01.85 US 688859**
**20.09.85 US 778036**
**25.09.85 US 780108**

(43) Date of publication of application:
**13.08.86 Bulletin 86/33**

(45) Publication of the grant of the patent:
**13.05.92 Bulletin 92/20**

(84) Designated Contracting States:
**AT BE CH DE FR GB IT LI LU NL SE**

(56) References cited:
**EP-A- 0 041 934**
**EP-A- 0 092 999**
**US-A- 3 972 328**

(73) Proprietor: **E.R. Squibb & Sons, Inc.**
**Lawrenceville-Princeton Road**
**Princeton, N.J. 08540-4000(US)**

(72) Inventor: **Cilento, Rudolfo D.**
**478 Hobart Rd.**
**North Brunwick New Jersey(US)**
Inventor: **Freeman, Frank M.**
**10 Brandon Rd.**
**Lawrenceville New Jersey(US)**

(74) Representative: **Thomas, Roger Tamlyn et al**
**D. Young & Co. 10 Staple Inn**
**London WC1V 7RD(GB)**

Rank Xerox (UK) Business Services
(3.08/2.19/2.0)

**Description**

This invention relates to wound dressings.

Kelly et al. in GB-A-1,550,614 disclose an absorbent material suitable for incorporation in sanitary towels, tampons and napkins comprising an absorbent starch derivative, preferably a cross-linked starch, distributed in a flexible resilient polyurethane foam.

W. R. Grace & Co. in GB-A-1,538,809 disclose a hydrophilic polyurethane foam in which a cross-linked carboxymethylcellulose and other water soluble or water dispersible materials such as sodium carboxymethylcellulose are incorporated.

Chen in US-A-3,972,328, Steer et al. in US-A-4,341,207, and Pawelchak et al. in US-A-4,538,603 and EP-A-92,999 disclose multi-layered dressings including an intermediate layer of semi-open cell polyurethane foam and an adhesive layer which contacts the wound and surrounding skin. Lindquist et al. in US-A-3,665,918 disclose a surgical tape or drape consisting of compressed polyurethane foam coated with a microporous pressure sensitive adhesive.

Richter et al. in US-A-3,566,871 disclose a hydrophilic polyurethane sponge for medical use in which the sponge pores contain a surfactant coating. McRae et al. in U.S. Patent US-A-3,978,855 disclose a polyurethane foam surgical dressing in which a surface of the foam is compressed and wherein the foam also contains a wetting agent. Lock in US-A-3,975,567 discloses a polyurethane foam surgical dressing in which one surface is rendered lyophilic by applying pressure and heat. Lazlo in EP-A-41,934 disclose a wound exudate absorbent product comprising a polyurethane foam having a water-insoluble hydrophilic polymer such as cross-linked dextran incorporated within a portion of the foam cells. Marsan in US-A-3,713,445 discloses an ostomy sealing ring consisting of an open cell polyurethane or polyethylene foam containing a gelatinous material such as karaya powder and glycerine. Various polyurethane foam dressings are disclosed by Robins in US-A-3,113,568, Bentov in US-A-3,157,178, Wheeler in U. S. Patent US-A-3,648,692, and Nowakowski in US-A-3,949,742.

This invention is directed to an occlusive wound dressing which includes a flexible closed cell polyurethane foam layer containing within the foam network from 5% to 50% by weight of the foam of one or more water dispersible, water swellable, and/or water absorbing agents. A pressure sensitive adhesive is applied or laminated onto one surface of the foam. In use, this adhesive surface contacts the wound site and the surrounding skin. A polymeric film may be laminated to or a skin may be formed on the opposite surface of the foam so as to protect the dressing.

More specifically the invention provides an occlusive wound dressing consisting essentially of a flexible closed cell polyurethane foam of from 10 to 100 mils (254 to 2540 $\mu$m) thickness and a density of from 10 pounds/cubic foot to 50 pounds/cubic foot (160 to 800 Kg/m$^3$) having distributed therein (i) from 10% to 50% by weight of the foam of one or more agents selected from pectin, gelatin, and sodium carboxymethylcellulose, (ii) from 5% to 22% by weight of said foam of a water insoluble starch-acrylonitrile graft copolymer, or (iii) from 12% to 48% by weight of said foam of a mixture of about 2 parts by weight of one or more of pectin, gelatin, and sodium carboxymethylcellulose and about 1 part by weight of a water insoluble starch-acrylonitrile graft copolymer, a polyurethane film or skin of from 0.5 to 3 mils (12.7 to 76.2 $\mu$m) thickness laminated to one surface of said foam, and a pressure sensitive adhesive of from 1 to 10 mils (25.4 to 254 $\mu$m) thickness which is either laminated to the other surface of said foam in a discontinuous pattern covering from 25% to 75% of the surface of said foam or is microporous.

Figure 1 is an overall view of a preferred embodiment of the wound dressing of this invention (greatly enlarged) including a sheet of release paper shown partially peeled away from the adhesive layers.

Figure 2 is a front view along line 2-2 of Figure 1 without the release paper.

Figure 3 is an overall view of another preferred embodiment of the wound dressing of this invention (greatly enlarged) including a sheet of release paper shown partially peeled away from the adhesive layer.

Figure 4 is a front view along line 4-4 of Figure 3 without the release paper.

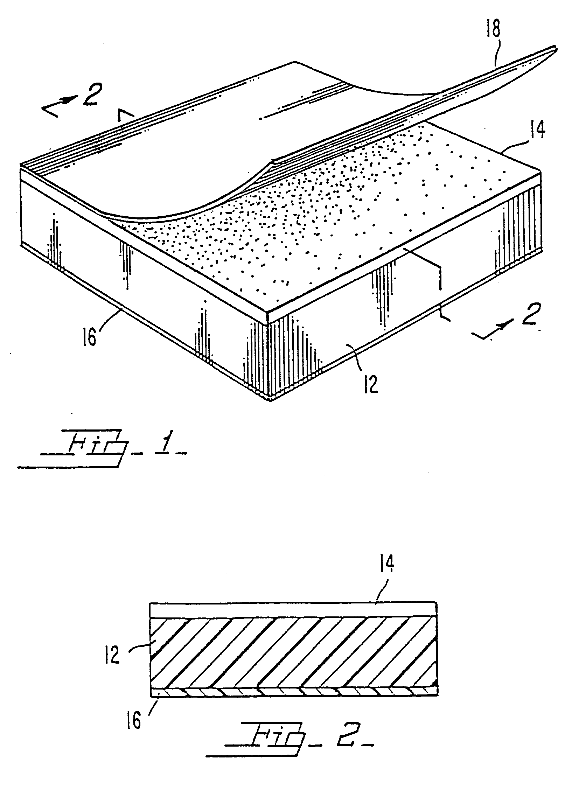

The occlusive dressing of this invention as shown in figures 1, 2, 3 and 4 includes a layer of closed cell polyurethane foam 12 and 12'. This foam layer contains from 5% to 50% by weight of the foam of one or more water dispersible or water swellable agents. A pressure sensitive adhesive 14 or 14' is coated or laminated to one surface of foam layer 12 or 12'. The adhesive 14 is applied as a discontinuous pattern on the surface of the foam so that portions of the foam surface remain uncovered. Preferably, the adhesive 14 is applied in the form of strips which may extend horizontally, vertically, or diagonally across the surface of the foam. Alternatively, adhesive 14 may be applied as a pattern of dots or squares across the surface of the foam. Adhesive 14 covers from 25% to 75% most preferably about 65%, of the surface area of foam layer 12. In use, the wound dressing is applied to the body by pressing so that adhesive layer 14 contacts the wound site and surrounding skin. The discontinuous pattern of pressure sensitive adhesive 14 permits the wound dressing to be firmly adhered to the body but enables the wound exudate to contact the foam wherein it reacts with the water dispersible, water swellable, and/or water absorbing agents within the foam network.

The pressure sensitive microporous adhesive layer 14' is coated or laminated to one surface of foam layer 12' as a continuous layer so that the entire surface of the foam is covered. The porosity of adhesive layer 14' enables wound exudate to migrate through this layer to contact the foam where it reacts with the water dispersible, water swellable, and/or water absorbing agents within the foam network.

The top surface of foam layer 12 or 12' when viewing the dressing placed on a wound is sealed so that soil and water cannot penetrate into the dressing. A thin film 16 or 16' of polymeric material such as polyurethane can be laminated to the top surface of foam layer 12 or 12' or the top surface of the foam can be treated with heat and/or pressure to flame laminate skin 16 or 16' on the surface of the foam. In another procedure, the skin 16 or 16' is cast and cured on a release liner and then the foam layer 12 or 12' is cast and formed directly on the skin.

The foam layer 12 or 12' is a flexible closed cell polyurethane foam formed from a polyester or a polyether and having one or more water dispersible, water swellable, and/or water absorbing agents distributed throughout the structure of the foam.

The foam should be of a density of from 10 pounds/cubic foot to 50 pounds/cubic foot (160 to 800 Kg/m$^3$).

Suitable water dispersible, water swellable, and/or water absorbing agents include one or more of sodium carboxymethylcellulose, pectin, gelatin, and substantially water insoluble starch-acrylonitrile graft copolymer such as that described in US-A-3,661,815 and that available commercially under the trademark Water Lock from the Grain Processing Corp.

The water dispersible, water swellable, and/or water absorbing agents are as follows. One or more of pectin, gelatin, and sodium carboxylmethylcellulose together at from 10% to 50% by weight of the foam and particularly an equal weight equal weight blend of pectin, gelatin, and sodium carboxymethylcellulose together at about 30% by weight of the foam. A water insoluble starch-acrylonitrile graft co-polymer at from 5% to 22% by weight of the foam and most preferably at about 12% by weight of the foam. A mixture of 2 parts by weight of one or more of pectin, gelatin, and sodium carboxymethylcellulose, most preferably an equal weight blend of pectin, gelatin and sodium carboxymethylcellulose, and one part by weight of a water insoluble starch-acrylonitrile graft copolymer. This mixture is present at from 12% to 48% by weight of the foam with the most preferred mixture being about 5% pectin, about 5% gelatin, about 5% sodium carboxymethylcellulose, and about 7.5% of a water insoluble starch-acrylonitrile graft copolymer by weight of the foam.

In addition, small amounts, i.e., in toto less than 5% by weight of the foam layer, of other agents may be included with the water dispersible, water swellable and/or water absorbing agents. For example, a pharmaceutically active ingredient such as an antibiotic or antimicrobial agent, an antiseptic agent such as povidone iodine, or an antiinflammatory agent such as hydrocortisone or triamcinolone acetonide maybe included. Other materials such as a deodorant, perfume agent, or antioxidant could be included. Also, substances conventionally employed in the manufacture of polyurethane foams to increase strength or flexibility can be included as long as they are safe and non-irritating to the human skin.

The flexible closed cell polyurethane foam 12 or 12' containing the water dispersible, water swellable and/or water absorbing agents plus any additional ingredients as described above can be prepared by either the conventional one-shot process or the conventional prepolymer process. In the one-shot process, a polyol, isocyanate and water are reacted substantially simultaneously with the water dispersible, water swellable and/or water absorbing agents and other ingredients suspended in the mixed reactants. In the prepolymer process, the polyol is reacted with enough polyisocyanate to form a prepolymer with isocyanate end groups plus excess isocyanate. This prepolymer mixture is then reacted with a blowing agent and a slurry of the water dispersible, water swellable, and/or water absorbing agents and other ingredients in a suitable solvent. After mixing, the resin can be dispersed by several methods. Typical methods are as an expandable liquid, as a spray of small droplets of mixed resin which adhere to surfaces and foam on these surfaces, or as a froth into which some gas has been mixed prior to exiting from the mixing head which causes the liquid mixture to froth as its pressure is decreased to atmospheric pressure. Additional materials may be used in order to form the polyurethane. For example, catalysts such as the tin catalysts, i.e., stannous octoate or dibutyl tin dilaurate, and an amine catalyst such as dimethylaminoethyl ether may be used to promote the reaction of the polyol and the isocyanate. A chemical blowing agent may be added which releases a gas due to the chemical decomposition of such blowing agent as a consequence of the heat of reaction or the external raising of the reaction temperature. Also, water may be used as a blowing agent since the isocyanate when reacted with water release carbon dioxide.

Typical polyurethane foam processes are described in more detail in Kirk-Othmer, Enc. of Chem. Tech., 3rd, (1979), Vol. 11, p 87 - 89 and Vol. 23, p 576 - 608, GB-A-1,550,614 and GB-A-1,538,809 noted above, and in US-A-3,004,934, 3,075,930, 3,441,523, 3,586,648, 3,753,933, 4,156,759, 4,197,372, 4,327,195, and 4,374,208.

In the preferred process the polyurethane foam 12 or 12' containing the water dispersible, water swellable, and/or water absorbing agents and any other optional agents is cast onto a polymeric film 16 or 16' which had previously been cast on silicone coated release paper. Alternatively, one surface of the foam 12 or 12' can be treated with heat and pressure to form skin 16 or 16'.

Pressure sensitive adhesive 14 may be of an acrylic based type for example a copolymer of isooctyl acrylate and acrylic acid as described by Ulrich in U.S. Patent RE 24,906 or a microporous acrylic adhesive as described by Copeland in US-A-3,121,021. Alternatively, pressure sensitive adhesive 14 may be a mixture of a rubbery elastomer preferably a mixture of low and medium molecular weight polyisobutylenes, one or more water dispersible agents preferably sodium carboxymethylcellulose and gelatin, a tackifier preferably a terpene resin, and a plasticizer preferably mineral oil as taught by Chen in US-A-3,972,328 or such a pressure sensitive adhesive in a microporous form as taught by Cilento et al in US-A-4,427,737.

Pressure sensitive adhesive 14 may be coated directly to foam layer 12 in the desired discontinuous pattern. Preferably, adhesive 14 is cast onto a sheet of silicone coated release paper 18 in the desired pattern and this coated sheet of paper is laminated (adhesive 14 contacting the foam) with pressure to the surface of foam 12 opposite polymeric film or skin 16. The resulting product is cut to the desired shape and packaged preferably with the release paper covering adhesive 14. At the time of use, the release paper is peeled away as shown in figure 1 and the wound dressing is applied to skin. If desired, the edges of the dressing may be more firmly attached to the body and prevented from curling by applying strips of a microporous adhesive tape.

Pressure sensitive microporous adhesive 14' may be of an acrylic type formed as taught by Copeland in US-A-3,121,021. Suitable acrylics include acrylic esters particularly those with four or more carbon atoms in the alcohol component such as n-butyl acrylate and/or 2-ethylhexyl acrylate. The acrylic adhesive may contain other comonomers such as vinyl acetate, acrylonitrile, styrene, ethyl acrylate, methyl methacrylate, $\alpha,\beta$-unsaturated carboxylic acids, esters, or half esters of unsaturated dicarboxylic acids. Terpolymers are also often used for this purpose. A discussion of pressure sensitive acrylic adhesives appears in Handbook of Adhesive, 2nd Edition, Skeist, pages 548-552.

Pressure sensitive microporous adhesive 14' may be formed from a mixture of a rubbery elastomer, one or more water dispersible agents, a tackifier, a plasticizer, and other optional ingredients. As taught by Cilento et al., in US-A-4,427,737, this microporous adhesive preferably contains of from about 35% to about 50% by weight of rubbery elastomer, from about 30% to about 60% by weight of water dispersible agents, and up to 35% by weight of one or more tackifiers, plasticizers, antioxidants and preservatives. Preferably, the elastomer is a mixture of low and medium molecular weight polyisobutylenes, the water dispersible agent is a mixture of a sodium carboxymethylcellulose and gelatin, the tackifier is a terpene resin, and the plasticizer is mineral oil.

The acrylic or rubbery elastomer adhesive can be made microporous by employing the solvent evaporation technique described by Copeland in US-A-3,121,021 and Cilento et al. in US-A-4,427,737. In this procedure, the solids are dispersed in a solvent such as heptane to form a slurry. The slurry is cast onto silicone coated release paper to the desired thickness and the material is then passed through a multi-zone drying tunnel so as to evaporate off the solvent.

Alternatively, the adhesive can be made microporous by forming an aqueous emulsion of the solids, adding a surfactant or a viscosity building agent such as sodium carboxymethylcellulose and incorporating air or other gaseous bubbles into the emulsion. This emulsion is then cast onto silicone coated release paper and dried by passing through a multi-zone drying tunnel.

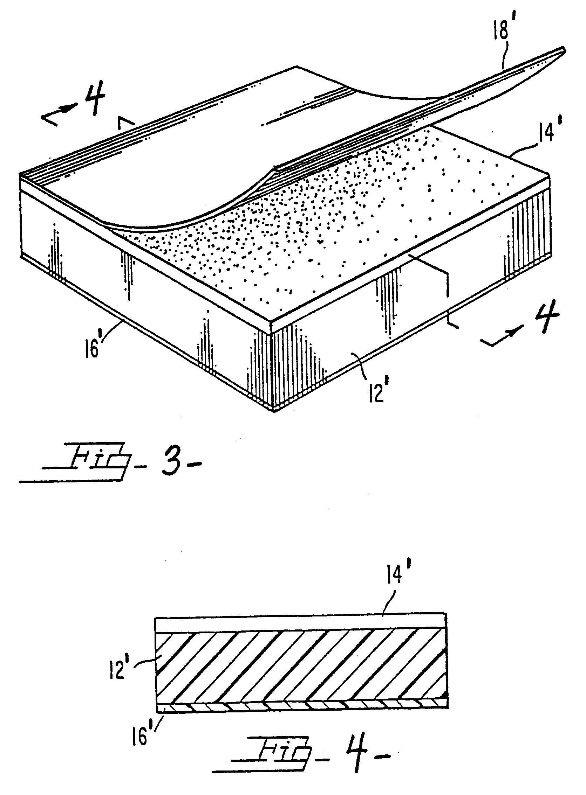

The pressure sensitive microporous adhesive layer including the silicone coated release paper is laminated (adhesive 14' contacting the foam) with pressure to the surface of foam 12' opposite polymeric film or skin 16'. The resulting product is cut to the desired shape and packaged preferably with the release paper covering adhesive 14'. At the time of use, the release paper is peeled away as shown in figure 3 and the wound dressing is applied to skin. If desired, the edges of the dressing may be more firmly attached to the body and prevented from curling by applying stripes of a microporous adhesive tape.

The foam layer 12 or 12' will be from 10 to 100 mils (254 to 2540 $\mu$m) thickness, most preferably from 20 to 30 mils (508 to 762 $\mu$m) thickness, pressure sensitive adhesive 14 or 14' will be from 1 to 10 mils (25.4 to 254 $\mu$m) thickness, most preferably from 2 to 4 mils (50.8 to 101.6 $\mu$m) thickness, and skin or polymeric film layer 16 or 16' will be from about 0.5 to 3 mils (12.7 to 76.2 $\mu$m) thickness, most preferably about 1 mils (25.4 $\mu$m) thickness.

Example 1

Polyurethane is cast onto a sheet of silicone release paper to give a film of about 1 mils (25.4 $\mu$m) thickness.

A flexible closed cell polyurethane foam is prepared by reacting appropriate amounts of a polyether polyol such as poly(oxyethylene) glycol, an isocyanate such as 4,4'-methylenebis(phenyl isocyanate), catalysts such as stannous octoate and dimethylaminoethyl ether and water (blowing agent) with sufficient starch-acrylonitrile graft copolymer (J-500 Water Lock). The starch-acrylonitrile graft copolymer is dispersed in the polyol. The other ingredients are mixed in a suitable vessel. The two mixtures are then intermixed at high speed in an in-line mixer, poured onto the polyurethane film, and allowed to cure to give a flexible closed cell polyurethane foam of about 25 mils (635 $\mu$m) thickness containing about 12% starch-acrylonitrile graft copolymer by weight of the foam.

An acrylic pressure sensitive adhesive such as a copolymer of isooctyl acrylate and acrylic acid in a 94:6 ratio is dispersed in an organic solvent. A portion of this slurry is poured onto a sheet of silicone coated release paper in horizontal strips of about 0.075 inch (1.9 mm) with about 0.05 inches (1.27 mm) between strips to leave a series of adhesive layers of about 2 mils (50.8 $\mu$m) thickness.

This series of adhesive layers is laminated to the closed cell polyurethane foam by gently compressing the adhesive to the foam by passing through pressure rollers.

The resulting wound dressing is cut to the desired size and packaged.

Examples 2 - 6

Following the procedure of Example 1 but employing the following ingredients on a weight percent basis within polyurethane foam layer 12 other dressings within the scope of this invention are obtained.

|  | Example | | | | |
|---|---|---|---|---|---|
|  | 2 | 3 | 4 | 5 | 6 |
| Pectin | 5 | 10 | - | 7 | 10 |
| Gelatin | 5 | 10 | - | 7 | - |
| Sodium carboxymethylcellulose | 5 | 10 | - | 6 | 10 |
| Starch-acrylonitrile graft copolymer (J-500 Water Lock) | 7.5 | - | 15 | - | 10 |

Example 7

Following the procedure of Example 1 but substituting for the acrylic adhesive a pressure sensitive adhesive of the following composition:

|  | Percent by weight |
|---|---|
| Polyisobutylene (Vistanex LM-MH) | 18.0 |
| Polyisobutylene (Vistanex L-100) | 20.0 |
| Terpene resin (Piccolyte) | 20.0 |
| Butylated hydroxytoluene | 0.5 |
| Mineral oil | 8.5 |
| Sodium carboxymethylcellulose | 18.0 |
| Gelatin | 15.0 |

A mixture of 0.31 kg. of polyisobutylene (Vistanex L-100), 0.28 kg. of sodium carboxymethylcellulose, and 0.23 kg. of gelatin is kneaded. This mixture is added to a solution of heptane containing 0.28 kg. of polyisobutylene (Vistanex LM-MH), 0.31 kg. of piccolyte resin, 8 g. of butylated hydroxytoluene, and 0.13 kg. of mineral oil to form an adhesive slurry. A portion of this slurry is poured onto a sheet of silicone release paper in horizontal strips of about 0.075 inch (1.9 mm) with about 0.05 inches (1.27 mm) between strips to leave a series of adhesive layers of about 4 mils (101.6 $\mu$m) thickness.

This series of adhesive layers is laminated to the closed cell polyurethane foam by gently compressing the adhesive layer to the foam by passing through pressure rollers.

The resulting wound dressing is cut to the desired size and packaged.

In a similar manner, the pressure sensitive adhesive of this Example can be employed with the polyurethane foams of Examples 2 to 6.

## Example 8

Polyurethane is cast onto a sheet of silicone release paper to give a film of about 1 mils (25.4 $\mu$m) thickness.

A flexible closed cell polyurethane foam is prepared by reacting appropriate amounts of a polyether polyol such as poly(oxyethylene) glycol, an isocyanate such as 4,4'-methylenebis(phenyl isocyanate), catalysts such as stannous octoate and dimethylaminoethyl ether and water (blowing agent) with sufficient starch-acrylonitrile graft copolymer (J-500 Water Lock). The starch-acrylonitrile graft copolymer is dispersed in the polyol. The other ingredients are mixed in a suitable vessel. The two mixtures are then intermixed at high speed in an in-line mixer, poured onto the polyurethane film, and allowed to cure to give a flexible closed cell polyurethane foam of about 25 mils (635 $\mu$m) thickness containing about 12% starch-acrylonitrile graft copolymer by weight of the foam.

An acrylic pressure sensitive microporous adhesive is prepared by homogenizing an aqueous emulsion (52% by weight solids) of 100 g. of Gelva RA2333 (water-based pressure sensitive acrylic resin from Monsanto), adding 0.7 g. of sodium carboxymethylcellulose, and continuing the homogenation to introduce air bubbles into the emulsion. This slurry is cast onto a sheet of silicone coated release paper at a wet thickness of about 10 mils (254 $\mu$m). This material is passed through a multi-zone drying tunnel with a residence time of 5 to 10 minutes. The resulting dry microporous adhesive is about 4 mils (101.6 $\mu$m) thick.

This microporous acrylic adhesive is laminated to the closed cell polyurethane foam by gently compressing the adhesive to the foam by passing through pressure rollers.

The resulting wound dressing is cut to the desired size and packaged.

## Examples 9 - 12

Following the procedure of Example 8 but employing the following ingredients on a weight percent basis within polyurethane foam layer 12' other dressings within the scope of this invention are obtained.

|  | Example | | | |
|---|---|---|---|---|
|  | 9 | 10 | 11 | 12 |
| Pectin | 10 | - | 7 | 10 |
| Gelatin | 10 | - | 7 | - |
| Sodium carboxymethylcellulose | 10 | - | 6 | 10 |
| Starch-acrylonitrile graft copolymer (J-500 Water Lock) | - | 15 | - | 10 |

## Example 13

Following the procedure of Example 8 but substituting for the microporous acrylic adhesive a pressure sensitive adhesive of the following composition:

|  | Percent by weight |
|---|---|
| Polyisobutylene (Vistanex LM-MH) | 18.0 |
| Polyisobutylene (Vistanex L-100) | 20.0 |
| Terpene resin (Piccolyte) | 20.0 |
| Butylated hydroxytoluene | 0.5 |
| Mineral oil | 8.5 |
| Sodium carboxymethylcellulose | 18.0 |
| Gelatin | 15.0 |

The above solids are dispersed in sufficient heptane to make a slurry containing 40% by weight solids. The slurry is appled via a knife-over-roller onto silicone coated release paper to a wet thickness of 10 mils (254 μm). The material is then passed through a multi-zone oven with a residence time of 5-10 minutes so as to reduce the solvent content to less than 1%. The resulting dry adhesive layer is 3 mils (76.2 μm) thick and has a porosity of about 5cc/sec/in$^2$ (0.775 cc/sec/cm$^2$). As the dry film emerges from the oven, it is laminated to the closed cell polyurethane foam prepared as described in Example 1 by gently compressing the adhesive to the foam by passing through pressure rollers.

The resulting wound dressing is cut to the desired size and packaged.

In a similar manner, the pressure sensitive microporous adhesive of this Example can be employed with the polyurethane foams of Examples 9 to 12.

**Claims**

1. An occlusive wound dressing consisting essentially of a flexible closed cell polyurethane foam (12, 12') of from 10 to 100 mils (254 to 2540 μm) thickness and a density of from 10 pounds/cubic foot to 50 pounds/cubic foot (160 to 800 Kg/m$^3$) having distributed therein (i) from 10% to 50% by weight of the foam (12, 12') of one or more agents selected from pectin, gelatin, and sodium carboxymethylcellulose, (ii) from 5% to 22% by weight of said foam (12) of a water insoluble starch-acrylonitrile graft copolymer, or (iii) from 12% to 48% by weight of said foam (12) of a mixture of 2 parts by weight of one or more of pectin, gelatin, and sodium carboxymethylcellulose and 1 part by weight of a water insoluble starch-acrylonitrile graft copolymer, a polyurethane film or skin (16, 16') of from 0.5 to 3 mils (12.7 to 76.2 μm) thickness laminated to one surface of said foam (12), and a pressure sensitive adhesive (14) of from 1 to 10 mils (25.4 to 254 μm) thickness which is either laminated to the other surface of said foam (12, 12') in a discontinuous pattern covering from 25% to 75% of the surface of said foam (12) or is microporous.

2. The dressing of claim 1 wherein said foam (12, 12') is from 20 to 30 mils (508 to 762 μm) thickness, said pressure sensitive adhesive (14, 14') is from 2 to 4 mils (50.8 to 101.6 μm) thickness, and said polyurethane film or skin (16, 16') is 1 mil (25.4 μm) thickness.

3. The dressing of claim 2 wherein said foam (12, 12') contains (i), said pectin, gelatin, and sodium carboxymethylcellulose being present in said foam (12, 12') at equal amounts on a weight percent basis.

4. The dressing of claim 3 wherein said foam (12, 12') contains 30% by weight of said mixture of pectin, gelatin, and sodium carboxymethylcellulose.

5. The dressing of claim 4 wherein said pressure sensitive adhesive (14, 14') is an acrylic adhesive.

6. The dressing of claim 4 wherein said pressure sensitive adhesive (14, 14') is a blend of low and medium molecular weight polyisobutylenes, sodium carboxymethylcellulose, gelatin, terpene resin, and mineral oil.

7. The dressing of claim 4 wherein said pressure sensitive adhesive (14) is applied to said foam (12) in a strip pattern and covers 65% of the surface area of said foam (12).

8. The dressing of claim 2 wherein said foam (12, 12') contains (ii), 12% by weight of a water insoluble starch-acrylonitrile graft copolymer being present.

9. The dressing of claim 8 wherein said pressure sensitive adhesive (14, 14') is an acrylic adhesive.

10. The dressing of claim 8 wherein said pressure sensitive adhesive (14, 14') is a blend of low and medium molecular weight polyisobutylenes, sodium carboxymethylcellulose, gelatin, terpene resin, and mineral oil.

11. The dressing of claim 8 wherein said pressure sensitive adhesive (14) is applied to said foam (12) in a strip pattern and covers 65% of the surface area of said foam (12).

**12.** The dressing of claim 2 wherein said foam (12, 12') contains (iii), 5% by weight of pectin, 5% by weight of gelatin, 5% by weight of sodium carboxymethylcellulose, and 7.5% by weight of water insoluble starch-acrylonitrile graft copolymer being present.

**13.** The dressing of claim 12 wherein said pressure sensitive adhesive (14, 14') is an acrylic adhesive.

**14.** The dressing of claim 12 wherein said pressure sensitive adhesive (14, 14') is a blend of low and medium molecular weight polyisobutylenes, sodium carboxymethylcellulose, gelatin, terpene resin, and mineral oil.

**Revendications**

**1.** Pansement occlusif composé essentiellement d'une mousse souple de polyuréthane à cellules fermées (12, 12') de 10 à 100 millipouces (254 à 2540 $\mu$m) d'épaisseur, ayant une masse volumique de 10 livres/pied cube à 50 livres/pied cube (160 à 800 kg/m$^3$), dans laquelle sont répartis (i) de 10 % à 50 %, en poids de la mousse(12, 12'), d'un ou plusieurs agents choisis entre la pectine, la gélatine et la carboxyméthylcellulose sodique, (ii) de 5 % à 22 %, en poids de ladite mousse (12), d'un copolymère-bloc d'amidon-acrylonitrile insoluble dans l'eau, ou (iii) de 12 % à 48 %, en poids de ladite mousse (12), d'un mélange de 2 parties en poids de pectine, de gélatine et/ou de carboxyméthylcellulose sodique, et de 1 partie en poids d'un copolymère-bloc d'amidon-acrylonitrile insoluble dans l'eau ; d'une pellicule ou d'une peau de polyuréthane (16, 16') de 0,5 à 3 millipouces (12,7 à 76,2 $\mu$m) d'épaisseur, plaquée sur l'une des surfaces de ladite mousse (12) ; et d'un adhésif (14) sensible à la pression, de 1 à 10 millipouces (25,4 à 254 $\mu$m) d'épaisseur, qui est soit plaqué sur l'autre surface de ladite mousse (12, 12') suivant un motif discontinu couvrant de 25 % à 75 % de la surface de ladite mousse (12), soit microporeux.

**2.** Pansement selon la revendication 1, dans lequel ladite mousse (12, 12') a une épaisseur de 20 à 30 millipouces (508 à 762 $\mu$m), ledit adhésif (14, 14') sensible à la pression est épais de 2 à 4 millipouces (50,8 à 101,6 $\mu$m), et ladite pellicule ou peau de polyuréthane (16, 16') est épaisse de 1 millipouce (25,4 $\mu$m).

**3.** Pansement selon la revendication 2, dans lequel ladite mousse (12, 12') contient (i), ladite pectine, ladite gélatine et ladite carboxyméthylcellulose sodique étant présentes dans ladite mousse (12, 12') en quantités égales en pourcentage pondéral.

**4.** Pansement selon la revendication 3, dans lequel ladite mousse (12, 12') contient 30 % en poids dudit mélange de pectine, de gélatine et de carboxyméthylcellulose sodique.

**5.** Pansement selon la revendication 4, dans lequel ledit adhésif (14, 14') sensible à la pression est un adhésif acrylique.

**6.** Pansement selon la revendication 4, dans lequel ledit adhésif (14, 14') sensible à la pression est un mélange de polyisobutylènes à bas poids moléculaire et à poids moléculaire moyen, de carboxyméthylcellulose sodique, de gélatine, de résine terpénique et d'huile minérale.

**7.** Pansement selon la revndication 4, dans lequel ledit adhésif (14) sensible à la pression est appliqué sur ladite mousse (12) sous forme de bandes et recouvre 65 % de la superficie de ladite mousse (12).

**8.** Pansement selon la revendication 2, dans lequel ladite mousse (12, 12') contient (ii), 12 % en poids d'un copolymère-bloc d'amidon-acrylonitrile insoluble dans l'eau étant présents.

**9.** Pansement selon la revendication 8, dans lequel ledit adhésif (14, 14') sensible à la pression est un adhésif acrylique.

**10.** Pansement selon la revendication 8, dans lequel ledit adhésif (14, 14') sensible à la pression est un mélange de polyisobutylènes à bas poids moléculaire et à poids moléculaire moyen, de carboxyméthylcellulose sodique, de gélatine, de résine terpénique et d'huile minérale.

**11.** Pansement selon la revendication 8, dans lequel ledit adhésif (14) sensible à la pression est appliqué sur ladite mousse (12) sous forme de bandes et recouvre 65 % de la superficie de ladite mousse (12).

**12.** Pansement selon la revendication 2, dans lequel ladite mousse (12, 12') contient (iii), 5 % en poids de pectine, 5 % en poids de gélatine, 5 % en poids de carboxyméthylcellulose sodique, et 7,5 % en poids de copolymère-bloc d'amidon-acrylonitrile insoluble dans l'eau étant présents.

**13.** Pansement selon la revendication 12, dans lequel ledit adhésif (14, 14') sensible à la pression est un adhésif acrylique.

**14.** Pansement selon la revendication 12, dans lequel ledit adhésif (14, 14') sensible à la pression est un mélange de polyisobutylènes à bas poids moléculaire et à poids moléculaire moyen, de carboxyméthylcellulose sodique, de gélatine, de résine terpénique et d'huile minérale.

**Patentansprüche**

**1.** Okklusiver Wundverband, bestehend im wesentlichen aus einem flexiblen Polyurethanschaum mit geschlossenen Zellen (12, 12') von 10 bis 100 mils (254 bis 2540 $\mu$m) Dicke und einer Dichte von 10 pounds/cubic foot bis 50 pounds/cubic foot (160 bis 800 kg/m$^3$), worin verteilt sind: (i) 10 bis 50 Gew.-% des Schaums (12, 12') eines oder mehrerer Mittel ausgewählt aus Pectin, Gelatine und Natriumcarboxymethylcellulose, (ii) 5 bis 22 Gew.-% des Schaums (12) eines wasserunlöslichen Stärke-Acrylnitril-Propfcopolymerisats oder (iii) 12 bis 48 Gew.-% des Schaums (12) eines Gemischs aus 2 Gew.-Teilen eines oder mehrerer der Mittel Pectin, Gelatine und Natriumcarboxymethylcellulose und 1 Gew.-Teil eines wasserunlöslichen Stärke-Acrylnitril-Pfropfcopolymerisats, einem Polyurethanfilm oder -häutchen (16, 16') von 0,5 bis 3 mils (12,7 bis 76,2 $\mu$m) Dicke, aufgebracht auf eine Oberfläche des Schaums (12), und eines Haftklebers (14) von 1 bis 10 mils (25,4 bis 254 $\mu$m) Dicke, der entweder auf die andere Oberfläche des Schaums (12, 12') in einem 25 bis 75 % der Oberfläche des Schaum (12) bedeckenden nicht-kontinuierlichen Muster aufgebracht ist oder mikroporös ist.

**2.** Verband nach Anspruch 1, wobei der Schaum (12, 12') 20 bis 30 mils (508 bis 762 $\mu$m) dick ist, der Haftkleber (14, 14') 2 bis 4 mils (50,8 bis 101,6 $\mu$m) dick ist und der Polyurethanfilm oder das -häutchen (16, 16') 1 mil (25,4 $\mu$m) dick ist.

**3.** Verband nach Anspruch 2, wobei der Schaum (12, 12') (i) enthält, wobei das Pectin, die Gelatine und Natriumcarboxymethylcellulose in dem Schaum (12, 12') in gleichen Mengen in Gewichtsprozent enthalten sind.

**4.** Verband nach Anspruch 3, wobei der Schaum (12, 12') 30 Gew.-% des Gemischs aus Pectin, Gelatine und Natriumcarboxymethylcellulose enthält.

**5.** Verband nach Anspruch 4, wobei der Haftkleber (14, 14') ein Klebemittel auf Acrylbasis ist.

**6.** Verband nach Anspruch 4, wobei der Haftkleber (14, 14') ein Gemisch aus Polyisobutylenen niederen und mittleren Molekulargewichts, Natriumcarboxymethylcellulose, Gelatine, Terpenharz und Mineralöl ist.

**7.** Verband nach Anspruch 4, wobei der Haftkleber (14) auf den Schaum (12) in einem Streifenmuster aufgebracht wird und 65 % der Fläche des Schaums (12) bedeckt.

**8.** Verband nach Anspruch 2, wobei der Schaum (12, 12') (ii) enthält, wobei 12 Gew.-% eines wasserunlöslichen Stärke-Acrylnitril-Pfropfcopolymerisats enthalten sind.

**9.** Verband nach Anspruch 8, wobei der Haftkleber (14, 14') ein Klebemittel auf Acrylbasis ist.

**10.** Verband nach Anspruch 8, wobei der Haftkleber (14, 14') ein Gemisch aus Polyisobutylenen niederen und mittleren Molekulargewichts, Natriumcarboxymethylcellulose, Gelatine, Terpenharz und Mineralöl ist.

11. Verband nach Anspruch 8, wobei der Haftkleber (14) auf den Schaum (12) in einem Streifenmuster aufgebracht wird und 65 % der Oberfläche des Schaums (12) bedeckt.

12. Verband nach Anspruch 2, wobei der Schaum (12, 12') (iii) enthält, wobei 5 Gew.-% Pectin, 5 Gew.-% Gelatine, 5 Gew.-% Natriumcarboxymethylcellulose und 7,5 Gew.-% eines wasserunlöslichen Stärke-Acrylnitril-Pfropfcopolymerisats enthalten sind.

13. Verband nach Anspruch 12, wobei der Haftkleber (14, 14') ein Klebemittel auf Acrylbasis ist.

14. Verband nach Anspruch 12, wobei der Haftkleber (14, 14') ein Gemisch aus Polyisobutylenen niederen und mittleren Molekulargewichts, Natriumcarboxymethylcellulose, Gelatine, Terpenharz und Mineralöl ist.

_FIG_ _1_

_FIG_ _2_

Fig. 3

Fig. 4